# EUROPEAN PATENT APPLICATION

(11) **EP 3 195 792 A1**
(43) Date of publication of application: **26.07.2017**
(21) Application number: 15842698.1
(22) Date of filing: 24.04.2015
(51) Int. Cl.: A61B 1/06, G02B 23/24

(54) **OPTICAL TRANSMITTER UNIT, METHOD FOR CONNECTING OPTICAL TRANSMITTER MODULE AND TRANSMISSION-SIDE OPTICAL CONNECTOR, AND ENDOSCOPE SYSTEM**

(30) Priority: 19.09.2014 JP 2014191851
(71) Applicant: Olympus Corporation, Tokyo 192-8507 (JP)
(72) Inventor: URAKAWA, Tsutomu, Tokyo 192-8507 (JP); KAWATA, Susumu, Tokyo 192-8507 (JP); KINOUCHI, Hideaki, Tokyo 192-8507 (JP)
(74) Representative: Schicker, Silvia
(86) International application number: PCT/JP2015/062564
(87) International publication number: WO 2016/042828

(57) **Abstract**

An optical transmitter unit, a method of connecting an optical transmitter module and a transmitter side optical connector, and an endoscope system which reduce a light loss in optical transmission are provided. An optical transmitter unit 10 is configured in such a manner that a transmitter side optical connector 20 and an optical transmitter module 30 are coupled and fixed by a fixing member. The transmitter side optical connector 20 includes a ferrule 22 that holds an optical fiber 21, and includes a flange portion 23. The optical transmitter module 30 includes a light emitting element 32 and a metal case 34. A connector side screw portion 24 is provided on the ferrule 22. A module side screw portion 37 formed of an elastic member and configured to be screwed with the connector side screw portion 24 is provided in a sleeve 35. When the transmitter side optical connector 20 and the optical transmitter module 30 are fixed by the fixing member, the module side screw portion 37 is elastically deformed by pressing force of the fixing member.

## Description

### Field

The present invention relates to an optical transmitter unit, a method of connecting an optical transmitter module and a transmitter side optical connector, and an endoscope system.

### Background

Conventionally, an endoscope system is used in the medical field when an organ of a subject such as a patient is observed. The endoscope system includes, for example, an endoscope and a processing device. The endoscope includes an insertion portion formed in a flexible elongated shape, a distal end of which is provided with an imaging sensor. The insertion portion is inserted into a body cavity of the subject. The processing device is coupled to the insertion portion via a cable and a connector to perform an image process on an in-vivo image captured by the imaging sensor. The processing device causes a display device to display the in-vivo image.

In recent years, an imaging sensor with a large number of pixels which enables a clearer image observation has been developed, and the use of the imaging sensor with a large number of pixels for the endoscope has been considered. In addition, the insertion portion is required to be reduced in diameter in consideration of easiness of introduction into the subject. Furthermore, in order to transmit a large volume of signals between the imaging sensor and the processing device at high speed while realizing the reduction in the diameter of the insertion portion, an optical transmission system that transmits a signal using laser light is employed in the endoscope system.

In the optical transmission system with the use of the laser light or the like, it is important to perform the transmission without reducing the light quantity of an optical signal emitted from a light emitting element such as a laser diode. As an example of such a technique, an optical connector and an aligning method have been disclosed in which a plurality of fitting portions and cutouts having different lengths in an axial direction are provided on an outer peripheral surface of a base fixed to a ferrule, the ferrule is rotated at an interval (90 degrees) of the provided fitting portions to adjust eccentricity (aligning), and fixation and coupling are performed (for example, refer to Patent Literature 1).

### Citation List

### Patent Literature

Patent Literature 1: JP H11-38276 A

### Summary

### Technical Problem

Although the adjustment of the eccentricity as well as the fixation and the coupling can be easily performed in Patent Literature 1, the eccentricity can only be adjusted every 90 degrees. Therefore, it is difficult to adjust the eccentricity so that the optical signal is transmitted using the maximum light quantity, which results in a significant light loss.

The present invention has been made in consideration of the above-mentioned circumstances, and an object thereof is to provide an optical transmitter unit, a method of connecting an optical transmitter module and a transmitter side optical connector, and an endoscope system which reduce a light loss in optical transmission.

### Solution to Problem

To solve the above-described problem and achieve the object, an optical transmitter unit according to the present invention includes: an optical transmitter module configured to convert an electric signal into an optical signal and transmit the optical signal; and a transmitter side optical connector connected to the optical transmitter module and configured to hold an end portion of an optical fiber transmitting the optical signal, wherein the transmitter side optical connector includes a ferrule configured to hold the optical fiber, and a flange portion provided at one end of the ferrule, the optical transmitter module includes a light emitting element, and a metal case configured to store the light emitting element, a connector side screw portion is provided on the ferrule or the flange portion, and a module side screw portion formed of an elastic member and configured to be screwed with the connector side screw portion is provided in a sleeve of the metal case into which the ferrule is inserted, and the module side screw portion is elastically deformed by pressing force of a fixing member when the transmitter side optical connector and the optical transmitter module are pressed and fixed by the fixing member.

Moreover, an optical transmitter unit according to the present invention includes: an optical transmitter module configured to convert an electric signal into an optical signal and transmit the optical signal; and a transmitter side optical connector connected to the optical transmitter module and configured to hold an end portion of an optical fiber transmitting the optical signal, wherein the transmitter side optical connector includes a ferrule configured to hold the optical fiber, and a flange portion provided at one end of the ferrule, the optical transmitter module includes a light emitting element, and a metal case configured to store the light emitting element, a connector side screw portion is provided on the ferrule or the flange portion, and a module side screw portion configured to be screwed with the connector side screw portion is provided in a sleeve of the metal case into which the ferrule is inserted, and the module side screw portion moves by means of pressing force of a fixing member when the transmitter side optical connector and the optical transmitter module are pressed and fixed by the fixing member.

Moreover, in the above-described optical transmitter unit according to the present invention, positioning markers are provided on an outer peripheral portion of the sleeve and an outer peripheral portion of the flange portion.

Moreover, a method of connecting the optical transmitter module and the transmitter side optical connector in one of the above-described optical transmitter units includes: a step of inserting the ferrule into the sleeve of the metal case; a light quantity measuring step of measuring a light quantity of emission light emitted from the light emitting element and transmitted by the optical fiber while rotating the transmitter side optical connector such that the connector side screw portion provided on the ferrule or the flange portion and the module side screw portion provided in the sleeve are screwed with each other; an eccentricity adjusting step of rotating the transmitter side optical connector to place the transmitter side optical connector and the optical transmitter module at a position where a maximum light quantity is obtainable based on a measurement result obtained in the light quantity measuring step; and a fixing step of pressing and fixing the transmitter side optical connector and the optical transmitter module using the fixing member.

Moreover, an endoscope system according to the present invention is inserted into a subject and captures an inside of the subject, the endoscope system including: a light source unit configured to emit light with which the inside of the subject is irradiated; an imaging unit configured to perform a photoelectric conversion on light reflected from the subject to generate an image signal; an optical transmission unit including: one of the above-described optical transmitter units configured to perform a photoelectric conversion on the image signal and transmit an optical signal obtained by the conversion using the optical fiber; an optical receiving module configured to perform a photoelectric conversion on the optical signal transmitted by the optical fiber; and a receiving side optical connector configured to connect the optical fiber to the optical receiving module; and an image processing unit configured to process the image signal based on a signal transmitted by the optical transmission unit.

Moreover, the above-described endoscope system according to the present invention includes a plurality of the optical transmission units, wherein grooves are formed on a flange of the transmitter side optical connector and a flange of the receiving side optical connector, and different types of the grooves are provided on the respective optical transmission units.

### Advantageous Effects of Invention

According to the present invention, screw portions are provided on a ferrule or a flange portion and in a sleeve into which the ferrule is inserted, and a transmitter side optical connector and an optical transmitter module can be placed at a position for the maximum light quantity while the screw portions are rotated so as to be screwed with each other. The transmitter side optical connector and the optical transmitter module can also be brought into close contact with each other. Therefore, a light loss at the time of transmission can be reduced.

### Brief Description of Drawings

FIG. 1 is a schematic view illustrating an overview configuration of an endoscope system according to a first embodiment of the present invention.
FIG. 2 is a view separately illustrating an optical transmitter module and a transmitter side optical connector that constitute an optical transmitter unit used in the endoscope system illustrated in FIG. 1.
FIG. 3 is a side view of the optical transmitter unit illustrated in FIG. 2.
FIG. 4 is a flowchart explaining a method of connecting the optical transmitter module and the transmitter side optical connector.
FIG. 5 is a diagram explaining an adjustment of eccentricity between the optical transmitter module and the transmitter side optical connector.
FIG. 6A is a view explaining the method of connecting the optical transmitter module and the transmitter side optical connector.
FIG. 6B is a view explaining the method of connecting the optical transmitter module and the transmitter side optical connector.
FIG. 7 is a view explaining a method of connecting an optical transmitter module and a transmitter side optical connector according to a first modification of the first embodiment of the present invention.
FIG. 8 is a view explaining a method of connecting an optical transmitter module and a transmitter side optical connector in an optical transmitter unit according to a second modification of the first embodiment of the present invention.
FIG. 9 is a side view of an optical transmitter unit according to a third modification of the first embodiment of the present invention.
FIG. 10 is a view separately illustrating an optical transmitter module and a transmitter side optical connector in an optical transmitter unit according to a second embodiment of the present invention.
FIG. 11 is a view explaining a method of connecting an optical transmitter module and a transmitter side optical connector in an optical transmitter unit according to a first modification of the second embodiment of the present invention.
FIG. 12 is a schematic view illustrating an overview configuration of an optical transmission unit according to a third embodiment of the present invention.
FIG. 13 is a view explaining a groove provided on a flange portion of a transmitter side optical connector according to the third embodiment of the present invention.
FIG. 14 is a view explaining a groove provided on a flange portion of a transmitter side optical connector according to a modification of the third embodiment of the present invention.

### Description of Embodiments

In the following description, as an embodiment for practicing the present invention (hereinafter referred to as the "embodiment"), an endoscope system will be described. The present invention is not limited by the embodiments. In the drawings, identical elements are provided with the same reference signs.

### (First Embodiment)

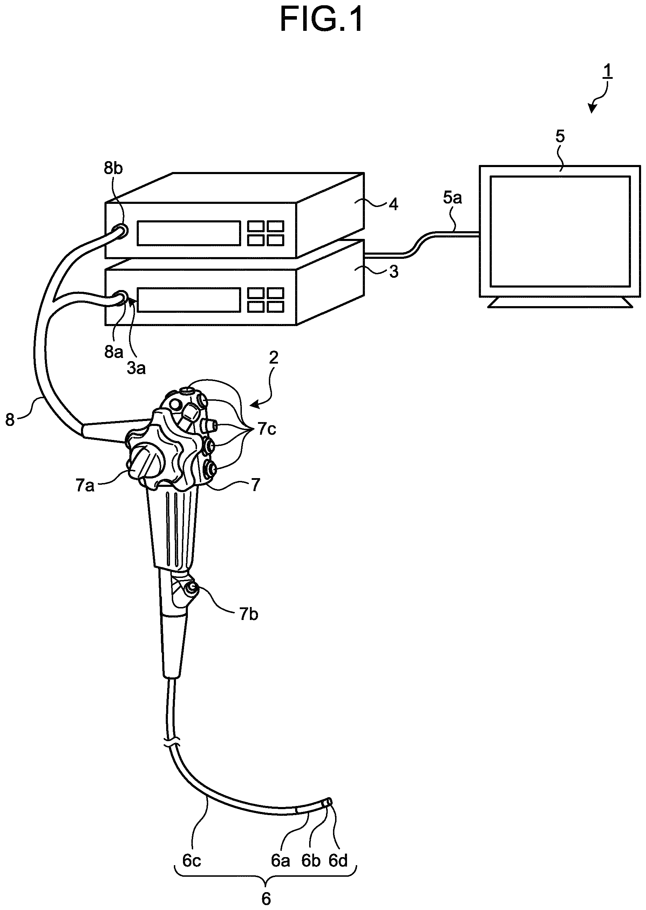

FIG. 1 is a schematic view illustrating an overview configuration of an endoscope system according to a first embodiment of the present invention. As illustrated in FIG. 1, an endoscope system 1 according to the present embodiment includes an endoscope 2, a processing device 3, a light source device 4, and a display device 5. The endoscope 2 is introduced into a subject and captures the inside of a body of the subject to generate an image signal of the inside of the subject. The processing device 3 performs a predetermined image process on the image signal captured by the endoscope 2, and controls each part of the endoscope system 1. The light source device 4 generates illumination light of the endoscope 2. The display device 5 displays an image of the image signal subjected to the image process by the processing device 3.

The endoscope 2 includes an insertion portion 6, an operating unit 7, and a flexible universal code 8. The insertion portion 6 is inserted into the subject. The operating unit 7 is located on a proximal end portion side of the insertion portion 6 and gripped by an operator. The universal code 8 extends from the operating unit 7.

The insertion portion 6 is realized with the use of an illumination fiber (light guide cable), an electric cable, and an optical fiber or the like. The insertion portion 6 includes a distal end portion 6a, a curve portion 6b, and a flexible pipe portion 6c. The distal end portion 6a includes an imaging unit in which an imaging sensor that captures the inside of the subject is incorporated. The curve portion 6b includes a plurality of curve pieces so as to be freely curved. The flexible pipe portion 6c is provided on a proximal end portion side of the curve portion 6b and has flexibility. The distal end portion 6a is provided with an illumination unit, an observation unit, an opening portion 6d, and an air/water supply nozzle (not illustrated). The illumination unit illuminates the inside of the subject through an illumination lens. The observation unit captures the inside of the subject. The opening portion 6d communicates with a processing tool channel.

The operating unit 7 includes a curve knob 7a, a treatment tool insertion portion 7b, and a plurality of switch units 7c. The curve knob 7a curves the curve portion 6b in an up-down direction and a left-right direction. A treatment tool such as living body forceps and a laser scalpel is inserted into a body cavity of the subject through the treatment tool insertion portion 7b. A peripheral device such as the processing device 3, the light source device 4, an air supply device, a water supply device, and a gas supply device is operated through the plurality of switch units 7c. The treatment tool inserted through the treatment tool insertion portion 7b passes through a treatment tool channel provided inside and comes out of the opening portion 6d at the distal end of the insertion portion 6.

The universal code 8 is configured with the use of an illumination fiber, an electric cable, and an optical fiber or the like. The universal code 8 branches at a proximal end thereof. An end portion of one of the branches is a connector 8a, and an end portion of the other is a connector 8b. The connector 8a is detachably attached to a connector 3a of the processing device 3. The connector 8b is detachably attached to the light source device 4. The universal code 8 propagates the illumination light emitted from the light source device 4 to the distal end portion 6a through the connector 8b, the operating unit 7, and the flexible pipe portion 6c. The universal code 8 transmits the image signal captured by the imaging unit provided in the distal end portion 6a to the processing device 3 by means of an optical transmitter unit to be described later.

The processing device 3 performs the predetermined image process on the image signal of the inside of the subject captured by the imaging unit of the distal end portion 6a of the endoscope 2. The processing device 3 controls each part of the endoscope system 1 based on various instruction signals transmitted from the switch units 7c of the operating unit 7 of the endoscope 2 through the universal code 8.

The light source device 4 is configured with the use of a light source that emits light and a condenser lens or the like. Under the control of the processing device 3, the light source device 4 emits the light from the light source and supplies, to the endoscope 2 coupled via the connector 8b and the illumination fiber of the universal code 8, the light as the illumination light for the inside of the subject that serves as an object.

The display device 5 is configured with the use of a display or the like in which liquid crystal or organic electro luminescence (EL) is used. The display device 5 displays, through a video cable 5a, various types of information including the image subjected to the predetermined image process by the processing device 3. Consequently, the operator can observe a desired position in the subject and determine the condition of the desired position by operating the endoscope 2 while watching the image (in-vivo image) displayed by the display device 5.

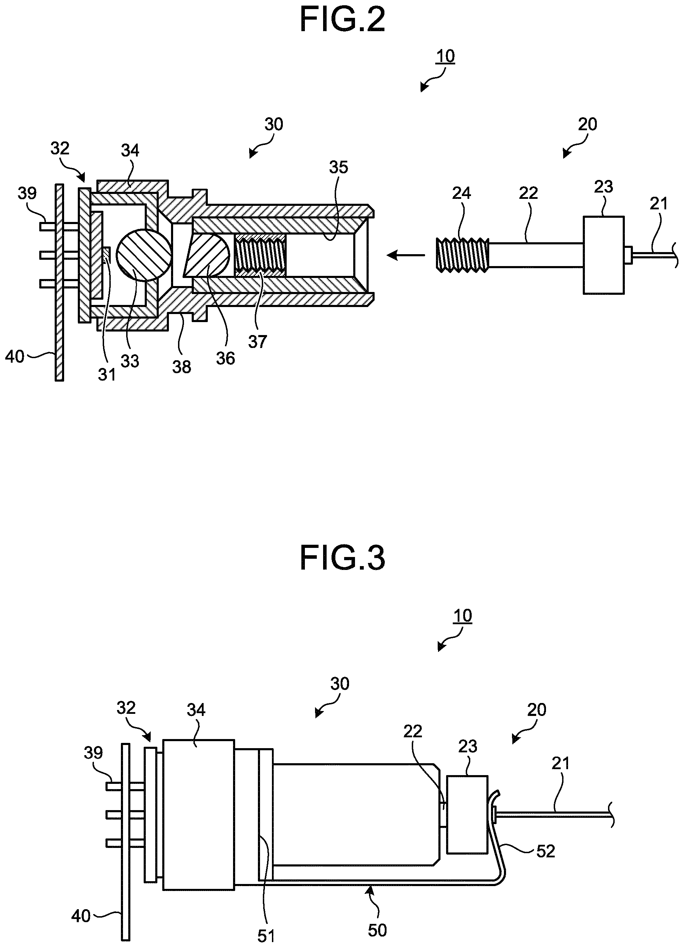

Next, in the endoscope 2 described in FIG. 1, the optical transmitter unit that transmits the image signal captured by the imaging unit to the processing device will be described. FIG. 2 is a view separately illustrating an optical transmitter module and a transmitter side optical connector that constitute the optical transmitter unit used in the endoscope system illustrated in FIG. 1. In FIG. 2, for an easy understanding, an optical transmitter module 30 is illustrated in a cross-sectional view, a transmitter side optical connector 20 is illustrated in a side view, and a fixing member is not illustrated. FIG. 3 is a side view of an optical transmitter unit 10 illustrated in FIG. 2.

The optical transmitter unit 10 is configured in such a manner that the transmitter side optical connector 20 and the optical transmitter module 30 are coupled by a fixing member 50 as illustrated in FIGS. 2 and 3. The optical transmitter unit 10 is arranged at the operating unit 7 or the insertion portion 6 of the endoscope 2.

The transmitter side optical connector 20 includes a ferrule 22 and a flange portion 23. The ferrule 22 holds an optical fiber 21. The flange portion 23 is provided at one end of the ferrule 22. In the ferrule 22 having a substantially columnar shape, a micro hole (not illustrated) that passes through a center of the columnar shape in an axial direction is provided. The optical fiber 21 is inserted into the micro hole, whereby the transmitter side optical connector 20 holds the optical fiber 21. The optical fiber 21 is inserted into the micro hole and exposed at an end surface (hereinafter referred to as a "distal end portion") of the ferrule 22 that is inserted into a sleeve 35. An end surface of the optical fiber 21 is polished in order to reduce a loss of the light quantity at an optical connection portion. A connector side screw portion 24 is formed on an outer peripheral portion of the ferrule 22 located on an insertion portion side. The flange portion 23 having a columnar shape that is concentric with the ferrule 22 is provided on an outer peripheral portion of the ferrule 22 located opposite to the distal end portion (hereinafter referred to as a "proximal end portion").

The optical transmitter module 30 includes a light emitting element 32 and a metal case 34. The metal case 34 stores and optically connects the light emitting element 32 and the ferrule 22. The light emitting element 32 is coupled to a flexible substrate 40 via a lead 39. The image signal captured by the imaging unit is transmitted to the light emitting element 32 through the flexible substrate 40, subjected to a photoelectric conversion, and emitted from a light emitting unit 31 as an optical signal. The optical signal emitted from the light emitting unit 31 is collected by a condenser lens 33 and a transparent glass body 36. The metal case 34 includes the sleeve 35 into which the ferrule 22 is inserted. A module side screw portion 37 that is screwed with the connector side screw portion 24 is provided in the vicinity of the transparent glass body 36 in the sleeve 35. The module side screw portion 37 is formed of an elastic deformable material such as rubber.

The ferrule 22 is inserted into the sleeve 35 of the optical transmitter module 30, the connector side screw portion 24 and the module side screw portion 37 are screwed with each other, and eccentricity is adjusted. After that, as illustrated in FIG. 3, the optical transmitter module 30 is fixed by the fixing member 50 that presses the ferrule 22 in the sleeve 35 of the metal case 34. The fixing member 50 is made of a metal material, and includes U-shaped holding portions 51 and 52 at both ends thereof. The holding portions 51 and 52 are fit with the optical transmitter unit 10 in an opening direction of the U shape, and fixed. The holding portion 51 is fit with a recessed portion 38 of the metal case 34, and the holding portion 52 is fit with a proximal end side of the flange portion 23 of the transmitter side optical connector 20. The length between the holding portion 51 and the holding portion 52 is designed to be shorter than the length from the recessed portion 38 to the proximal end portion of the flange portion 23. Therefore, when the fixing member 50 is fit with the optical transmitter unit 10, the fixing member 50 performs the fixation while pressing the ferrule 22 in the sleeve 35.

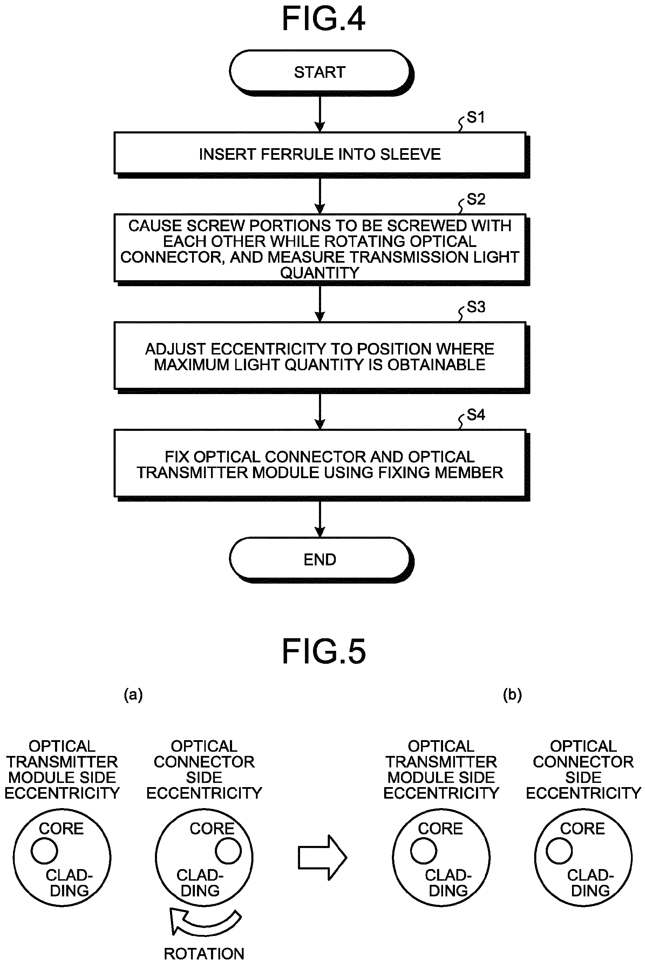

Next, a connecting method for the optical transmitter unit 10 will be described. FIG. 4 is a flowchart explaining a method of connecting the optical transmitter module 30 and the transmitter side optical connector 20.

First, the flange portion 23 of the transmitter side optical connector 20 is directly gripped, or the transmitter side optical connector 20 is gripped using a jig attached to the flange portion 23, and the ferrule 22 is inserted into the sleeve 35 of the optical transmitter module 30 (step S1).

After the ferrule 22 is inserted into the sleeve 35, the transmitter side optical connector 20 is rotated, and the connector side screw portion 24 is screwed with the module side screw portion 37. At this time, the light is emitted from the light emitting unit 31, and the light quantity transmitted by the optical fiber 21 is measured on a proximal end side of the optical fiber 21 (step S2).

In the transmitter side optical connector 20, the eccentricity might occur between an outer diameter center of the ferrule 22 and a core center of the optical fiber 21 inserted into the micro hole during the manufacturing process. Similarly, the eccentricity might also occur between a center of the light emitting unit 31 and a center of the sleeve 35 in the optical transmitter module 30.

FIG. 5 is a diagram explaining the adjustment of the eccentricity between the optical transmitter module 30 and the transmitter side optical connector 20. FIG. 5(a) is a state before the adjustment of the eccentricity, and FIG. 5(b) is a state after the adjustment of the eccentricity. In FIGS. 5(a) and 5(b), the eccentricity is illustrated on a large scale for an easy understanding.

In a case where the eccentric transmitter side optical connector 20 and the eccentric optical transmitter module 30 are optically connected, no problem occurs when a direction of the eccentricity of the transmitter side optical connector 20 is the same as that of the optical transmitter module 30. However, in a case where the directions are different from each other as illustrated in FIG. 5(a), the transmission light quantity is significantly reduced if the transmitter side optical connector 20 and the optical transmitter module 30 are connected as they are. In a case where the transmitter side optical connector 20 and the optical transmitter module 30 that are eccentric in the different directions are connected, for example, the transmitter side optical connector 20 is rotated as illustrated by an arrow in FIG. 5(a), whereby the direction of the eccentricity between the transmitter side optical connector 20 and the optical transmitter module 30 can be adjusted as illustrated in FIG. 5(b), and the light loss can be reduced. In the first embodiment, since the connector side screw portion 24 and the module side screw portion 37 are screwed with each other to adjust the eccentricity, the transmission light quantity can be sequentially detected, and a position where the maximum light quantity is obtainable can be detected.

After the transmitter side optical connector 20 is rotated, and the connector side screw portion 24 is screwed with the module side screw portion 37, the transmitter side optical connector 20 is rotated in an opposite direction until the transmitter side optical connector 20 reaches a position for the maximum light quantity, whereby the eccentricity between the transmitter side optical connector 20 and the optical transmitter module 30 is adjusted (step S3).

After the eccentricity between the transmitter side optical connector 20 and the optical transmitter module 30 is adjusted, the transmitter side optical connector 20 and the optical transmitter module 30 are fixed by the fixing member 50 (step S4).

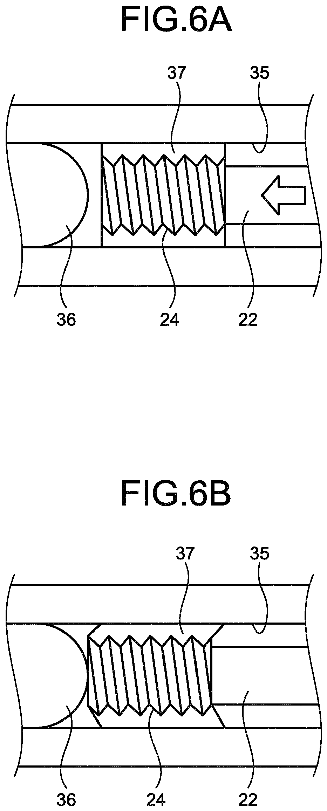

In a case where the light quantity is measured in step S2, and the maximum light quantity is obtained when the distal end portion of the ferrule 22 is located at the rearmost end of the module side screw portion 37 as illustrated in FIG. 6A, the adjustment of the eccentricity is finished at the position in FIG. 6A. However, the light loss occurs since a space exists between the transparent glass body 36 and the distal end portion of the ferrule 22. In the first embodiment, the module side screw portion 37 is formed of the elastic member. Therefore, when the fixing member 50 is fit with the optical transmitter unit 10 in FIG. 6A subjected to the adjustment of the eccentricity, pressing force is exerted in a direction illustrated by an arrow in FIG. 6A, and the module side screw portion 37 is elastically deformed as illustrated in FIG. 6B. Consequently, the transparent glass body 36 and the distal end portion of the ferrule 22 can be brought into contact with each other and connected, and the light loss can be reduced.

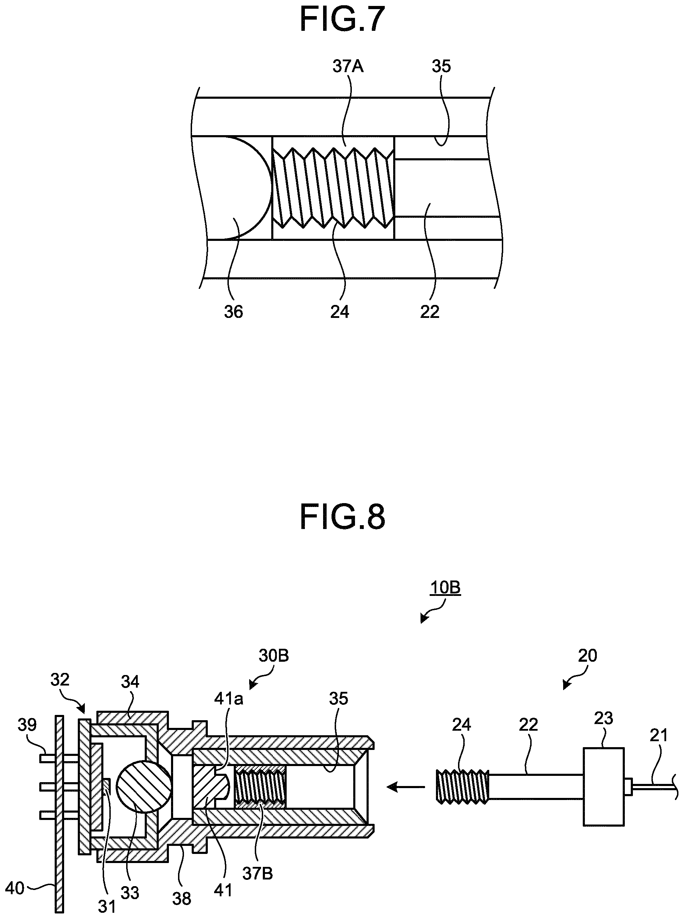

Alternatively, the module side screw portion may move through the inside of the sleeve 35 instead of being elastically deformed by the pressing force caused by the fixing member 50. FIG. 7 is a view explaining a method of connecting an optical transmitter module and a transmitter side optical connector according to a first modification of the first embodiment of the present invention. In the first modification, a module side screw portion 37A is formed at a position apart from the transparent glass body 36 in the same way as that of the first embodiment illustrated in FIG. 6A. However, the module side screw portion 37A is pressed by the fixing member 50 and moves in a direction toward the transparent glass body 36 together with the ferrule 22. In the first modification, the pressing force of the fixing member 50 only needs to be set to be larger than frictional force between the sleeve 35 and the module side screw portion 37A. Consequently, the transparent glass body 36 and the distal end portion of the ferrule can be brought into contact with each other and connected, and the light loss can be reduced.

In addition, a similar effect can be obtained in the optical transmitter module that uses a stub 41 in place of the transparent glass body 36. FIG. 8 is a view explaining a method of connecting an optical transmitter module 30B and the transmitter side optical connector 20 according to a second modification of the first embodiment of the present invention. In the second modification, the optical transmitter module 30B and the transmitter side optical connector 20 are optically connected using the stub 41. A groove portion 41a is formed on an outer peripheral side of the stub 41 that is in contact with the sleeve 35. In the same way as the module side screw portion 37A of the first modification, a module side screw portion 37B can move in a direction toward the stub 41 together with the ferrule 22 when pressed by the fixing member 50, and go into the groove portion 41a. Even in a case where the maximum light quantity is obtained when the distal end portion of the ferrule 22 is located before the rearmost end of the module side screw portion 37B, since the groove portion 41a is formed on the stub 41, the distal end portion of the ferrule 22 and the stub 41 can be brought into contact with each other and connected, and the light loss can be reduced.

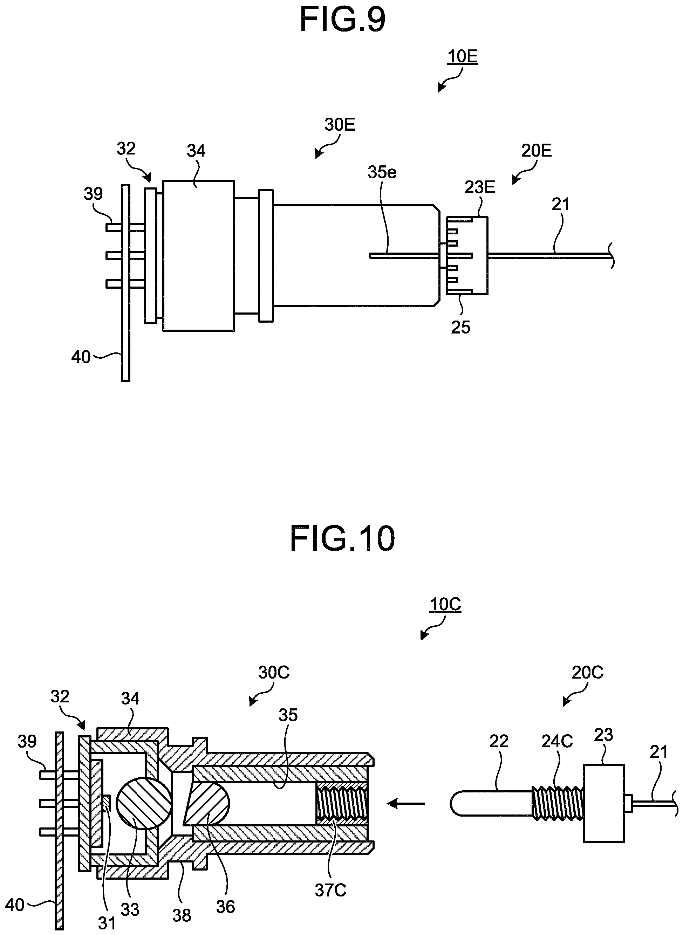

In the first embodiment, the adjustment of the eccentricity between the optical transmitter module and the transmitter side optical connector is performed by causing the screw portions provided in the sleeve and on the surface of the ferrule to be fit with each other. Alternatively, the adjustment of the eccentricity can be easily performed by providing a positioning marker on each of an outer peripheral portion of the sleeve and an outer peripheral portion of the flange portion. FIG. 9 is a side view of an optical transmitter unit according to a third modification of the first embodiment of the present invention. In the optical transmitter unit 10E according to the third modification, a marker 35e and a marker 25 are provided on the outer peripheral portion of the sleeve and the outer peripheral portion of a flange portion 23E, respectively. The adjustment of the eccentricity is facilitated by providing the marker 25 and the marker 35e.

### (Second Embodiment)

FIG. 10 is a view separately illustrating an optical transmitter module and a transmitter side optical connector in an optical transmitter unit according to a second embodiment of the present invention. In an optical transmitter unit 10C according to the second embodiment of the present invention, a module side screw portion 37C is provided on an insertion opening side of the sleeve 35 for the ferrule 22, and a connector side screw portion 24C is provided on a side of the ferrule 22 located close to the flange portion 23.

In the second embodiment as well, the ferrule 22 is inserted into the sleeve 35, a transmitter side optical connector 20C is rotated, and the connector side screw portion 24C is screwed with the module side screw portion 37C, whereby the transmitter side optical connector 20C is adjusted to reach a position for the maximum light quantity. After that, an optical transmitter module 30C and the transmitter side optical connector 20C are pressed and fixed by the fixing member, and the transparent glass body 36 and the distal end portion of the ferrule 22 are brought into contact with each other and connected, whereby the light loss can be reduced. In the same way as the module side screw portion 37 of the first embodiment, the module side screw portion 37C may be an elastic member, and the transparent glass body 36 and the distal end portion of the ferrule 22 may be connected in contact with each other by means of the elastic deformation of the module side screw portion 37C. Alternatively, in the same way as the module side screw portion 37A of the first modification of the first embodiment, the module side screw portion 37C may be configured to move through the inside of the sleeve 35 by means of the pressing force. A similar effect can be obtained when the transparent glass body 36 is replaced by the stub 41.

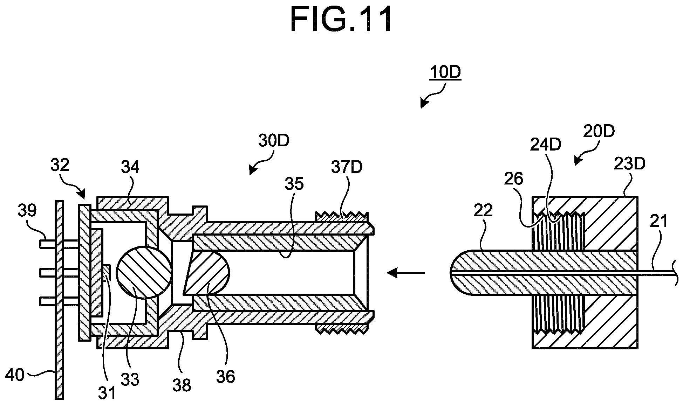

In addition, the module side screw portion provided on the insertion opening side of the sleeve 35 for the ferrule 22 may be provided on the outer peripheral portion of the sleeve 35, not in the sleeve 35. FIG. 11 is a cross-sectional view explaining a method of connecting an optical transmitter module 30D and a transmitter side optical connector 20D according to a first modification of the second embodiment of the present invention. In an optical transmitter unit 10D according to the first modification of the second embodiment, a flange portion 23D is formed to be thicker in diameter than the sleeve 35, and provided with, at a distal end side of the ferrule 22, an insertion portion 26 into which the sleeve 35 of the metal case 34 is inserted. A connector side screw portion 24D is provided in the insertion portion 26. In the first modification of the second embodiment as well, the eccentricity can be adjusted to obtain the maximum light quantity, and the light loss can be reduced. In the same way as the module side screw portion 37 of the first embodiment, a module side screw portion 37D may be an elastic member, and the transparent glass body 36 and the distal end portion of the ferrule 22 may be connected in contact with each other by means of the elastic deformation of the module side screw portion 37D. Alternatively, in the same way as the module side screw portion 37A of the first modification of the first embodiment, the module side screw portion 37D may be configured to move on the surface of the sleeve 35 by means of the pressing force.

### (Third Embodiment)

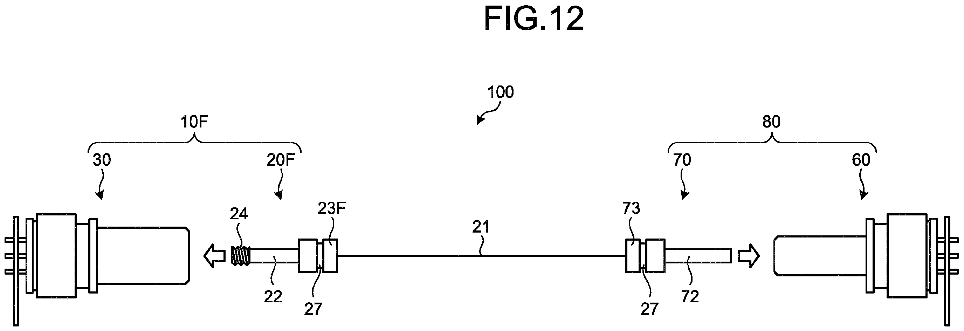

In an endoscope of a third embodiment, the transmission of the image signal is performed using a plurality of optical transmission units. FIG. 12 is a schematic view illustrating an overview configuration of the optical transmission unit according to the third embodiment of the present invention.

An optical transmission unit 100 includes an optical transmitter unit 10F, the optical fiber 21, and an optical receiving unit 80. The optical transmitter unit 10F is installed at the operating unit or the insertion portion of the endoscope, and the optical receiving unit 80 is installed in the processing device. The optical transmitter unit 10F is configured in such a manner that the optical transmitter module 30 and a transmitter side optical connector 20F illustrated in FIG. 12 are coupled and fixed by the fixing member. The optical transmitter module 30 has a configuration similar to that of the optical transmitter module 30 of the first embodiment. The transmitter side optical connector 20F includes a flange portion 23F in place of the flange portion 23 of the transmitter side optical connector 20 of the first embodiment. The flange portion 23F includes a groove 27 on an outer peripheral portion thereof. Since the flange portion 23F serves as a grip portion, a corner portion that constitutes the groove 27 is preferably rounded. The groove 27 may be provided over the entire periphery of the flange portion 23F, or may be partially provided.

The optical receiving unit 80 is configured in such a manner that an optical receiving module 60 and a receiving side optical connector 70 illustrated in FIG. 12 are coupled and fixed by a fixing member. The optical receiving module 60 performs a photoelectric conversion on the optical signal transmitted by the optical fiber 21. Although the optical receiving module 60 includes a sleeve into which a ferrule 72 of the receiving side optical connector 70 to be described later is inserted, a module side screw portion is not formed in the sleeve. The receiving side optical connector 70 includes the ferrule 72 and a flange portion 73. The ferrule 72 holds the optical fiber 21. The flange portion 73 includes a groove 27 and is provided at one end of the ferrule 72. In the third embodiment, the flange portion 73 has the same shape as the flange portion 23F of the transmitter side optical connector 20F. However, the ferrule 72 is different from the ferrule 22 in that a distal end portion of the ferrule 72 does not include a connector side screw portion. Alternatively, in order to perform the adjustment in the optical receiving unit 80 so that the light quantity received at the optical receiving module 60 is equal to or more than the lowest receiving sensitivity, a connector side screw portion and a module side screw portion similar to those of the optical transmitter unit 10C may be respectively provided on the ferrule 72 and inside a metal case of the optical receiving module, and may be fit with each other.

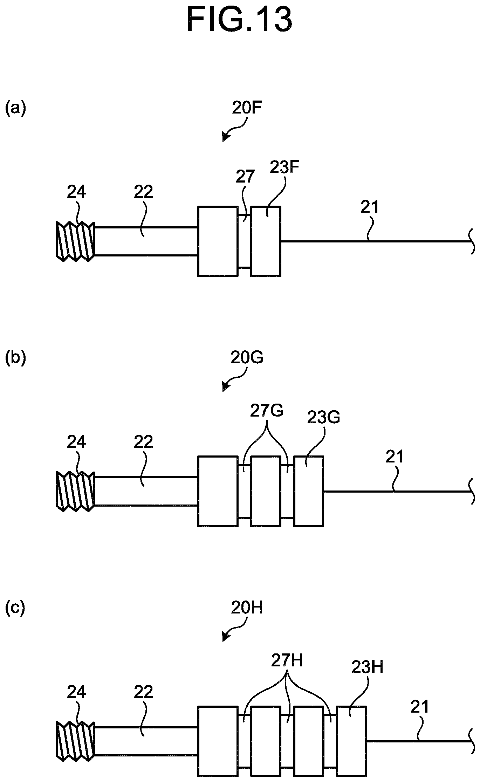

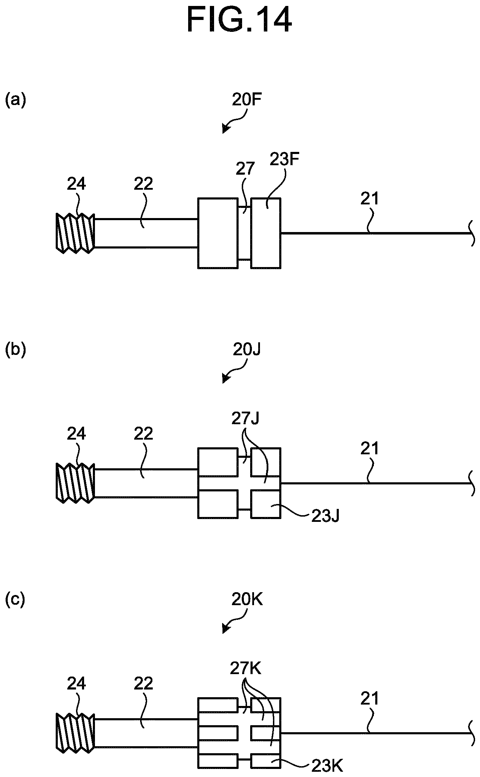

In a case where the image signal is transmitted using the plurality of optical transmission units, an insertion error might occur since the optical transmission units cannot be identified. In the third embodiment, therefore, different types of grooves are provided on the respective optical transmission units in order to identify the plurality of optical transmission units. For example, in a case where three types of optical transmission units are used, the optical transmission units can be identified by changing the number of grooves formed on the flange portions of each optical transmission unit as illustrated in FIGS. 13 and 14. In FIG. 12, the transmitter side optical connector 20F having the one groove 27 and the receiving side optical connector 70 having the one groove 27 constitute the optical transmission unit 100. In an optical transmission unit that uses a transmitter side optical connector 20G having two grooves 27G illustrated in FIG. 13(b), a receiving side optical connector having two grooves 27G is used, and in an optical transmission unit that uses a transmitter side optical connector 20H having three grooves 27H illustrated in FIG. 13(c), a receiving side optical connector having three grooves 27H is used, whereby the optical transmission units can be identified. The same applies to a case where transmitter side optical connectors having grooves 27, 27J, and 27K shaped as illustrated in FIG. 14 are used. The shapes of the grooves are not limited to those illustrated in FIGS. 13 and 14. Reference Signs List

1 ENDOSCOPE SYSTEM
2 ENDOSCOPE
3 PROCESSING DEVICE
4 LIGHT SOURCE DEVICE
5 DISPLAY DEVICE
6 INSERTION PORTION
6a DISTAL END PORTION
6b CURVE PORTION
6c FLEXIBLE PIPE PORTION
6d OPENING PORTION
7 OPERATING UNIT
7a CURVE KNOB
7b TREATMENT TOOL INSERTION PORTION
7c SWITCH UNIT
8 UNIVERSAL CODE
8a, 8b CONNECTOR
10, 10B, 10C, 10D, 10E OPTICAL TRANSMITTER UNIT
20, 20C, 20D, 20E, 20F, 20G, 20H, 20J, 20K TRANSMITTER SIDE OPTICAL CONNECTOR
21 OPTICAL FIBER
22, 72 FERRULE
23, 23D, 23E, 73 FLANGE PORTION
24, 24C, 24D CONNECTOR SIDE SCREW PORTION
25, 35e MARKER
26 INSERTION PORTION
27 GROOVE
30, 30B, 30C, 30D, 30E OPTICAL TRANSMITTER MODULE
31 LIGHT EMITTING UNIT
32 LIGHT EMITTING ELEMENT
33 CONDENSER LENS
34 METAL CASE
35 SLEEVE
36 TRANSPARENT GLASS BODY
37, 37A, 37B, 37C, 37D MODULE SIDE SCREW PORTION
38 RECESSED PORTION
39 LEAD
40 FLEXIBLE SUBSTRATE
41 STUB
50 FIXING MEMBER
51, 52 HOLDING PORTION
60 OPTICAL RECEIVING MODULE
70 RECEIVING SIDE OPTICAL CONNECTOR
80 OPTICAL RECEIVING UNIT
100 OPTICAL TRANSMISSION UNIT

## Claims

1. An optical transmitter unit comprising:
an optical transmitter module configured to convert an electric signal into an optical signal and transmit the optical signal; and
a transmitter side optical connector connected to the optical transmitter module and configured to hold an end portion of an optical fiber transmitting the optical signal, wherein
the transmitter side optical connector includes
a ferrule configured to hold the optical fiber, and
a flange portion provided at one end of the ferrule,
the optical transmitter module includes
a light emitting element, and
a metal case configured to store the light emitting element,
a connector side screw portion is provided on the ferrule or the flange portion, and a module side screw portion formed of an elastic member and configured to be screwed with the connector side screw portion is provided in a sleeve of the metal case into which the ferrule is inserted, and
the module side screw portion is elastically deformed by pressing force of a fixing member when the transmitter side optical connector and the optical transmitter module are pressed and fixed by the fixing member.

2. An optical transmitter unit comprising:
an optical transmitter module configured to convert an electric signal into an optical signal and transmit the optical signal; and
a transmitter side optical connector connected to the optical transmitter module and configured to hold an end portion of an optical fiber transmitting the optical signal, wherein
the transmitter side optical connector includes
a ferrule configured to hold the optical fiber, and
a flange portion provided at one end of the ferrule,
the optical transmitter module includes
a light emitting element, and
a metal case configured to store the light emitting element,
a connector side screw portion is provided on the ferrule or the flange portion, and a module side screw portion configured to be screwed with the connector side screw portion is provided in a sleeve of the metal case into which the ferrule is inserted, and
the module side screw portion moves by means of pressing force of a fixing member when the transmitter side optical connector and the optical transmitter module are pressed and fixed by the fixing member.

3. The optical transmitter unit according to claim 1 or 2, wherein
positioning markers are provided on an outer peripheral portion of the sleeve and an outer peripheral portion of the flange portion.

4. A method of connecting the optical transmitter module and the transmitter side optical connector in the optical transmitter unit according to any one of claims 1 to 3, the method comprising:
a step of inserting the ferrule into the sleeve of the metal case;
a light quantity measuring step of measuring a light quantity of emission light emitted from the light emitting element and transmitted by the optical fiber while rotating the transmitter side optical connector such that the connector side screw portion provided on the ferrule or the flange portion and the module side screw portion provided in the sleeve are screwed with each other;
an eccentricity adjusting step of rotating the transmitter side optical connector to place the transmitter side optical connector and the optical transmitter module at a position where a maximum light quantity is obtainable based on a measurement result obtained in the light quantity measuring step; and
a fixing step of pressing and fixing the transmitter side optical connector and the optical transmitter module using the fixing member.

5. An endoscope system for being inserted into a subject and capturing an inside of the subject, the endoscope system comprising:
a light source unit configured to emit light with which the inside of the subject is irradiated;
an imaging unit configured to perform a photoelectric conversion on light reflected from the subject to generate an image signal;
an optical transmission unit including:
the optical transmitter unit according to any one of claims 1 to 3 configured to perform a photoelectric conversion on the image signal and transmit an optical signal obtained by the conversion using the optical fiber;
an optical receiving module configured to perform a photoelectric conversion on the optical signal transmitted by the optical fiber; and
a receiving side optical connector configured to connect the optical fiber to the optical receiving module; and
an image processing unit configured to process the image signal based on a signal transmitted by the optical transmission unit.

6. The endoscope system according to claim 5, comprising a plurality of the optical transmission units, wherein
grooves are formed on a flange of the transmitter side optical connector and a flange of the receiving side optical connector, and different types of the grooves are provided on the respective optical transmission units.
